# EUROPEAN PATENT APPLICATION

(11) **EP 4 092 028 A1**
(43) Date of publication of application: **23.11.2022**
(21) Application number: 21767152.8
(22) Date of filing: 10.03.2021
(51) Int. Cl.: C07D 473/18, A61K 31/522, A61P 1/04

(54) **CRYSTAL OF HYPOXANTHINE COMPOUND**

(30) Priority: 11.03.2020 JP 2020041454
(71) Applicant: KISSEI PHARMACEUTICAL CO., LTD., Matsumoto-shi Nagano 399-8710 (JP)
(72) Inventor: KIJIMA Yoshiro, Azumino-shi, Nagano 399-8304 (JP); TAKEUCHI Hideki, Azumino-shi, Nagano 399-8304 (JP); TAKIGAWA Yasushi, Azumino-shi, Nagano 399-8304 (JP); KAWABE Yusuke, Azumino-shi, Nagano 399-8304 (JP)
(74) Representative: Isarpatent
(86) International application number: PCT/JP2021/009406
(87) International publication number: WO 2021/182491

(57) **Abstract**

As a drug substance, a crystal having good physical properties is preferable. However, the crystal form that is most excellent as a drug substance may vary with the compound. In general, it is difficult to predict a crystal form of a drug substance having good physical properties, and it is required to variously examine each compound. Therefore, an object of the present invention is to provide a crystal having good physical properties as a drug substance for a novel compound.

The present invention relates to a crystal of the compound represented by the following formula (I) useful for the treatment of an inflammatory bowel disease.

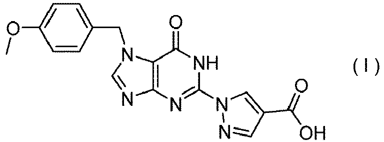

## Description

### [Technical Field]

The present invention relates to a crystal of a hypoxanthine compounds useful as a medicament.

More particularly, the present invention relates to a crystal of a hypoxanthine compound which has a prolyl hydroxylase inhibitory effect and which is useful as an agent for the treatment of an inflammatory bowel disease such as ulcerative colitis.

### [Background Art]

Inflammatory bowel diseases (IBDs) are chronic diseases in which inflammation and ulcers are caused in the intestinal mucosa due to excessive immune response. IBDs include, for example, ulcerative colitis and Crohn's disease.

Ulcerative colitis is a large intestine disease causing diffuse non-specific inflammation of uncertain cause. Large intestine mucosa is ulcerated, and erosion or ulcers may be caused in mucosa. Ulcerative colitis may be divided into "active phase" in which bloody stool, erosion, ulcers and the like are observed and "remission phase" in which the observations of the active phase disappear. Long-term treatment is required because relapse and remission are often repeated in the course.

For the treatment of ulcerative colitis, a 5-aminosalicylic acid formulation (5-ASA) is first used as a standard agent. However, it has been reported that the effectiveness of 5-ASA is approximately 46 to 64% and that patients with remission by administration of 5-ASA are no more than 29 to 45%. When the effect of 5-ASA is not observed, a steroid is used. Immunosuppressive agents, TNF-α antibodies and the like are sometimes used for the treatment of ulcerative colitis in addition to those medicaments. However, all the medicaments have problems such as side effects and necessity for careful administration. Therefore, a therapeutic agent having a novel mode of action for ulcerative colitis is desired.

It has been known that expression of genes associated with barrier function of gastrointestinal epithelium is induced by hypoxia-inducible factor 1α (HIF-1α) in a pathological condition of IBD. HIF-1α is one of the subtypes of hypoxia-inducible factor α (HIF-α). HIF-α is stabilized in a hypoxic environment (Hypoxia), and then it activates the transcription of various genes in response to hypoxia. In contrast, the proline residues of HIF-α are hydrolyzed by prolyl hydroxylases (PHDs) in an oxygen-rich environment (Normoxia), and then the HIF-α is degraded via the proteasomal pathway.

Three subtypes are known for PHDs, namely PHD1, PHD2 and PHD3. AKB-4924 is known as a PHD inhibitor. It has been reported that AKB-4924 has a PHD2 inhibitory effect and stabilizes HIF-1α in large intestine tissues (Non-patent literature 1). Furthermore, AKB-4924 has an improvement effect in TNBS induced colitis model.

In contrast, PHD inhibitors, such as Roxadustat and Daprodustat, have a hematopoietic effect and have been developed as therapeutic agents for anemia (Non-patent literature 2). Thus, it is important to avoid systemic effects such as a hematopoietic effect when a PHD inhibitor is used as a therapeutic agent for IBD.

For example, quinazolinone compounds are described in Patent literature 1 and pyrazolopyrimidine compounds are described in Patent literature 2 as PHD inhibitors. Compounds including hypoxanthine are described or illustrated in Patent literatures 3 to 7 and Non-patent literature 3. However, the crystals of the hypoxanthine compound of the present invention are not described in the above literatures.

### Citation List

### [Patent Literature]

Patent literature 1: WO 2010/093727
Patent literature 2: U.S. Published Application No. 2015/ 0239889
Patent literature 3: U.S. Published Application No. 2015/ 0368247
Patent literature 4: U.S. Published Application No. 2013/ 0165426
Patent literature 5: U.S. Published Application No. 2010/ 0029671
Patent literature 6: U.S. Published Application No. 2006/ 0258651
Patent literature 7: U.S. Published Application No. 2010/ 0120761

### [Non-patent literature]

Non-patent literature 1: Ellen Marks et al., "Inflamm. Bowel. Dis.", 2015, Vol. 21, No. 2, pp. 267-275
Non-patent literature 2: Mun Chiang Chan et al., "Molecular Aspects of Medicine", 2016, Vol. 47-48, pp. 54-75
Non-patent literature 3: Takashi Goi et al., "Synlett", 2018, Vol. 29, No. 14, pp. 1867-1870

### Summary of the Invention

### [Problems to Be Solved by the Invention]

The present applicant developed the compound represented by the following formula (I) (hereinafter, referred to as "compound 1") as a novel compound which has a PHD2 inhibitory effect, and they filed a patent application for the invention (PCT/JP2019/035995). 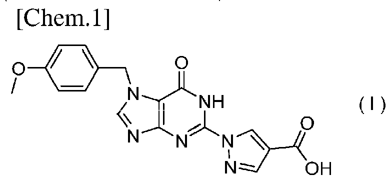 (Chemical name: 1-(7-(4-Methoxybenzyl)-6-oxo-6,7-dihydro-1H-purin-2-yl)-1H-pyrazole-4-carboxylic acid)

As a drug substance, a crystal having good physical properties is preferable. However, the crystal form that is most excellent as a drug substance may vary with the compound. In general, it is difficult to predict a crystal form of a drug substance having good physical properties, and it is required to variously examine each compound. Therefore, an object of the present invention is to provide a crystal having good physical properties as a drug substance for the compound 1, which is a novel compound.

### [Means for Solving the Problems]

The present invention relates to a crystal of the compound 1 which has a PHD2 inhibitory effect and which is useful for the treatment of an inflammatory bowel disease. That is, the present invention relates to the following [1] to [7] and the like.
[1] A crystal of the compound represented by the following formula (I): 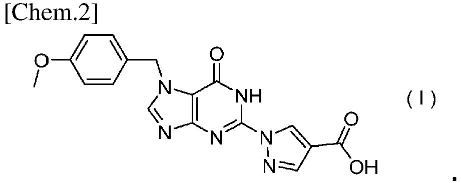
[2] The crystal according to the above [1] which is a crystal of the compound having a subset of the peaks at diffraction angles (2*θ* (°)) selected from the group consisting of the following (i) to (iv) in a powder X-ray diffraction:
   (i) peaks of 6.5±0.2 and 13.5±0.2 (crystal form I);
   (ii) peaks of 5.1±0.2 and 16.9±0.2 (crystal form II);
   (iii) peaks of 5.8±0.2 and 17.7±0.2 (crystal form III); and
   (iv) peaks of 5.0±0.2 and 5.6±0.2 (crystal form IV).
[3] The crystal according to the above [1] which is a crystal of the compound having a subset of the peaks at diffraction angles (2*θ* (°)) selected from the group consisting of the following (i) to (iv) in a powder X-ray diffraction:
   (i) peaks of 6.5±0.2 and 16.2±0.2 (crystal form I);
   (ii) peaks of 5.1±0.2 and 10.8±0.2 (crystal form II);
   (iii) peaks of 5.8±0.2 and 25.4±0.2 (crystal form III); and
   (iv) peaks of 5.0±0.2, 15.2±0.2 and 26.4±0.2 (crystal form IV).
[4] The crystal according to the above [1] which is a crystal of the compound having peaks at diffraction angles (2*θ* (°)) of 5.8±0.2, 8.4±0.2, 13.6±0.2, 17.0±0.2, 17.4±0.2, 17.7±0.2, 25.4±0.2 and 25.8±0.2 in a powder X-ray diffraction.
[5] A pharmaceutical composition comprising the crystal according to any one of the above [1] to [4] and a pharmaceutical additive.
[6] The pharmaceutical composition according to the above [5] which is a pharmaceutical composition for use in the treatment of an inflammatory bowel disease.
[7] The pharmaceutical composition according to the above [6], wherein the inflammatory bowel disease is ulcerative colitis or Crohn's disease.

In an embodiment, the present invention relates to a method for treating an inflammatory bowel disease, comprising administering a necessary amount of the pharmaceutical composition according to the above [5] to a patient.

In an embodiment, the present invention relates to a use of the crystal of the compound according to any one of the above [1] to [4] for manufacturing a pharmaceutical composition for use in the treatment of an inflammatory bowel disease.

In an embodiment, the present invention is the crystal according to the above [1] which is a crystal of the compound having a subset of the peaks at diffraction angles (2*θ* (°)) selected from the group consisting of the following (i) to (iv) in a powder X-ray diffraction:
(i) peaks of 6.5±0.2, 13.5±0.2, 16.2±0.2 and 17.5±0.2 (crystal form I);
(ii) peaks of 5.1±0.2, 10.8±0.2, 16.4±0.2 and 16.9±0.2 (crystal form II);
(iii) peaks of 5.8±0.2, 13.6±0.2, 17.7±0.2 and 25.4±0.2 (crystal form III); and
(iv) peaks of 5.0±0.2, 5.6±0.2, 15.2±0.2 and 26.4±0.2 (crystal form IV).

In an embodiment, the present invention is the crystal according to the above [1] which is a crystal of the compound having a subset of the peaks at diffraction angles (2*θ* (°)) in a powder X-ray diffraction and heat absorption, wherein the subset of the peaks and the heat absorption are selected from the group consisting of the following (i) to (iv):
(i) peaks of 6.5±0.2, 13.5±0.2, 16.2±0.2 and 17.5±0.2 and the endothermic peak top around 300°C (crystal form I);
(ii) peaks of 5.1±0.2, 10.8±0.2, 16.4±0.2 and 16.9±0.2 and the endothermic peak top around 301°C (crystal form II);
(iii) peaks of 5.8±0.2, 13.6±0.2, 17.7±0.2 and 25.4±0.2 and the endothermic peak top around 294°C (crystal form III); and
(iv) peaks of 5.0±0.2, 5.6±0.2, 15.2±0.2 and 26.4±0.2 and the endothermic peak top around 301°C (crystal form IV);

### Effect of the Invention

The crystal of the compound of the present invention has good physical properties as a drug substance.

### Brief Description of Drawings

Fig. 1 to Fig. 4 are powder X-ray diffraction diagrams of crystals. The vertical axis shows the diffraction intensity (Counts). The horizontal axis shows the diffraction angle (2*θ*(°)).
Fig. 5 to Fig. 8 are thermogravimetry differential thermal analysis charts (TG-DTA measurement diagrams) of crystals. The vertical axis (left) shows the mass change (%) in a thermogravimetric (TG) curve. The vertical axis (right) shows the heat flux (µV) in a differential thermal analysis (DTA) curve. The horizontal axis shows the temperature (°C).

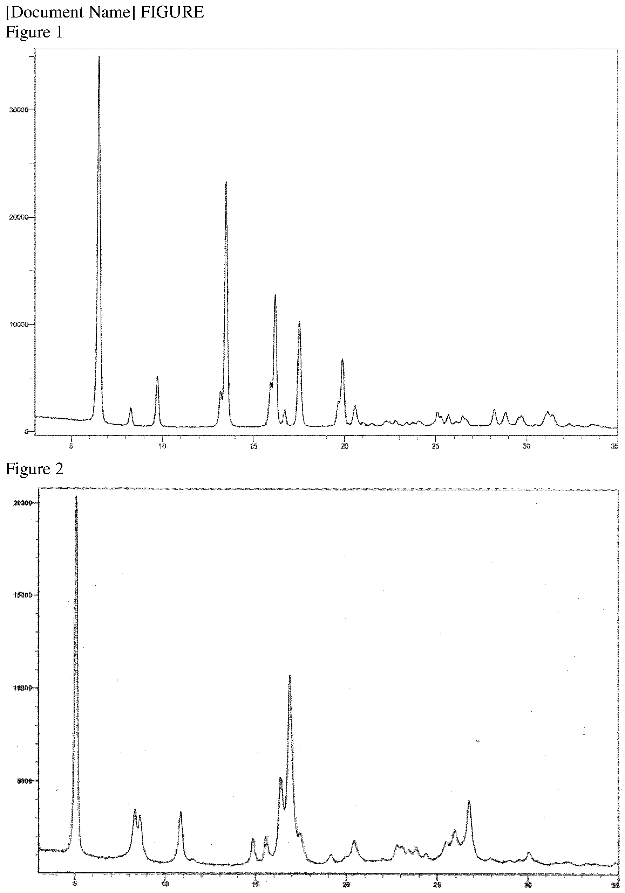

[Fig. 1] A powder X-ray diffraction diagram of crystal form I of the compound 1
[Fig. 2] A powder X-ray diffraction diagram of crystal form II of the compound 1

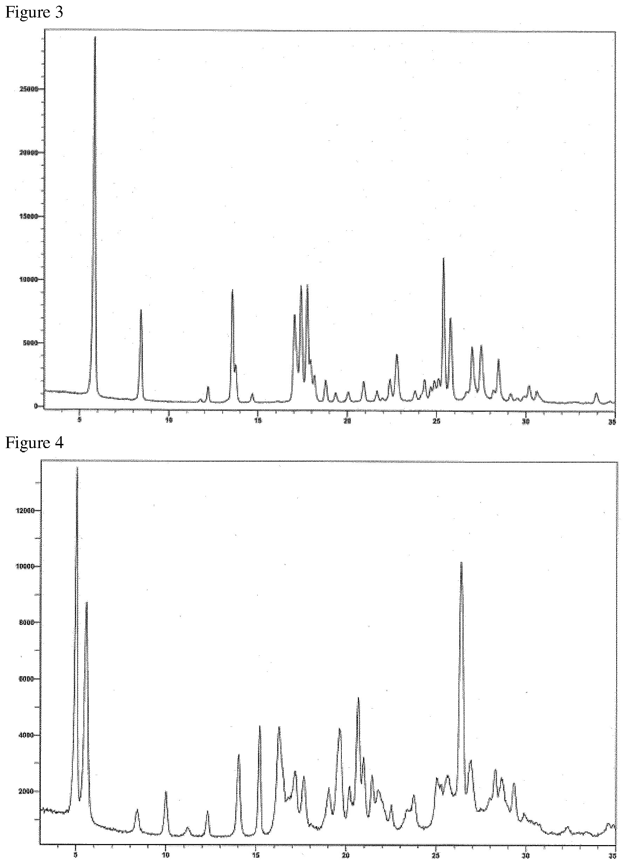

[Fig. 3] A powder X-ray diffraction diagram of crystal form III of the compound 1
[Fig. 4] A powder X-ray diffraction diagram of crystal form IV of the compound 1

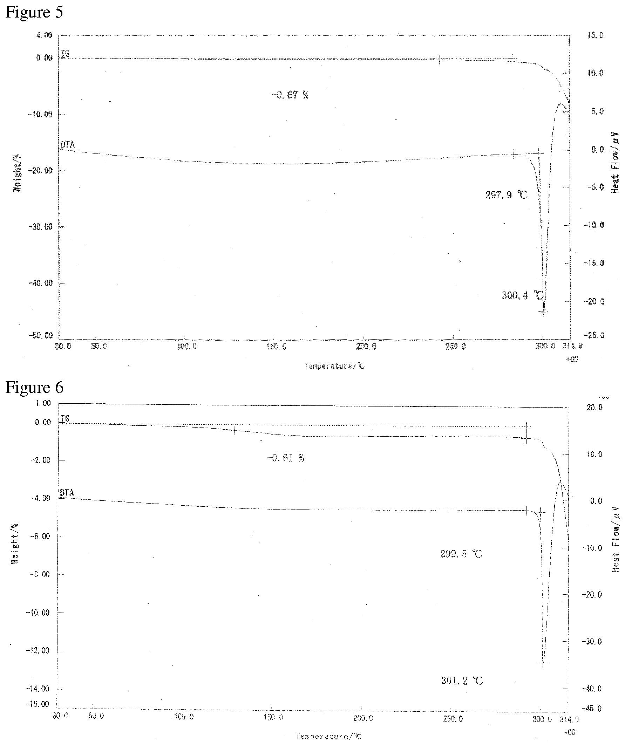

[Fig. 5] A TG-DTA measurement diagram of crystal form I of the compound 1
[Fig. 6] A TG-DTA measurement diagram of crystal form II of the compound 1

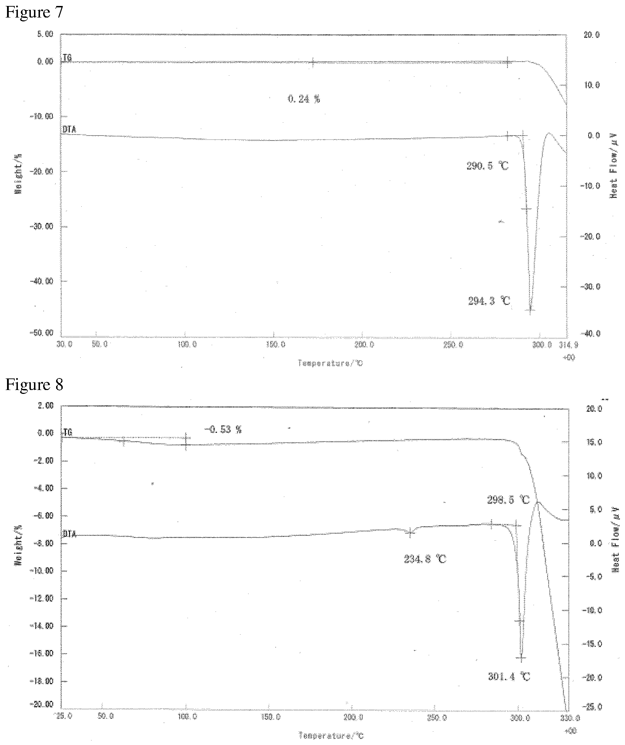

[Fig. 7] A TG-DTA measurement diagram of crystal form III of the compound 1
[Fig. 8] A TG-DTA measurement diagram of crystal form IV of the compound 1

### [Mode for Carrying out the Invention]

Hereinafter, embodiments of the present invention are described in more detail.

In the present invention, each term has the following meaning unless otherwise specified.

The following abbreviations in the description, figures and tables have the following meanings, respectively.
CDCl₃: deuterated chloroform
DMSO: dimethylsulfoxide
NMP: 1-methyl-2-pyrrolidinone
THF: tetrahydrofuran
TNBS: trinitrobenzene sulfonic acid
Pd₂(dba)₃: tris(dibenzylideneacetone)dipalladium(0)
tBuXPhos: 2-di-tert-butylphosphino-2',4',6'-triisopropylbiphenyl
amino-silica gel: aminopropylated silica gel
ODS column chromatography: octadecyl-silylated silica gel column chromatography
Structure: structural formula
Physical data: physical data
IC₅₀: concentration required for 50% inhibition
FITC: Fluorescein isothiocyanate
¹H-NMR: hydrogen nuclear magnetic resonance spectrum
DMSO-d6: dimethylsulfoxide-d6
MS: mass spectrometry
ESI_APCI: multiionization using electrospray ionization-atmospheric pressure chemical ionization

In the present invention, "as a drug substance, good physical properties" means, for example, that a crystal is physicochemically stable or chemically stable in the solid stability test shown in Test Example 4.

The crystal of the compound 1 of the present invention also includes a solvate thereof with a pharmaceutically acceptable solvent such as water or ethanol.

In the compound 1 of the present invention, part of the atoms may be replaced with corresponding isotopes. The present invention includes compounds in which atoms are replaced with these isotopes. Examples of the isotopes include isotopes of a hydrogen atom, a carbon atom, a chlorine atom, a fluorine atom, an iodine atom, a nitrogen atom, an oxygen atom, a phosphorus atom and a sulfur atom represented by ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, ³²P and ³⁵S. In an embodiment, a compound in which part of the hydrogen atoms of the compound 1 are replaced with ²H (D: deuterium atoms) can be illustrated.

The compound 1 of the present invention in which part of the atoms are replaced with isotopes can be prepared by a similar method to the method for manufacturing described below using a commercial isotope-introduced building block.

The compound 1 of the present invention has an excellent PHD2 inhibitory effect and thus can be used as a therapeutic agent for IBD (see, Nature Reviews Drug Discovery, 2014, 13, pp. 852-869). In the present invention, the phrase "IBD" includes, for example, ulcerative colitis, Crohn's disease, intestinal Behcet disease, infectious enteritis, radiation enteritis, drug-induced enteritis, ischemic enteritis, mesenteric phlebosclerosis (phlebosclerotic colitis), obstructive colitis and enteritis due to collagen disease. Preferably, the compound 1 of the present invention can be used as a therapeutic agent for ulcerative colitis or Crohn's disease (see, Inflamm. Bowel. Dis., 2015, 21 (2), pp. 267-275).

In the present invention, the phrase "treatment" includes the meanings of "prevention". The treatment of ulcerative colitis includes, for example, the meanings of "prevention of relapse" and "maintenance of remission".

The therapeutic effects on colitis of the compound 1 of the present invention can be determined according to the method described in Test Example 2 or well-known methods in the technical field. For example, the effects can also be determined according to the method described in Biol. Pharm. Bull., 2004, 27 (10), pp.1599-1603 and the like or similar methods thereto.

In an embodiment, the compound 1 of the present invention is a PHD2 inhibitor that acts specifically on the large intestine tissue to limit the off-target effects of stabilization of HIF-α. The term "acts specifically on the large intestine tissue" means, for example, that the concentration of the compound is high in the large intestine tissue compared to that in the blood and that the compound exerts a therapeutic effect on large intestine without systemic effects (for example, hematopoietic effect) (see, Test Examples 2 and 3).

The pharmaceutical composition of the present invention is used in various dosage forms depending on the usage. As such dosage forms, for example, powders, granules, fine granules, dry syrups, tablets, capsules, injections, liquids, ointments, suppositories, poultices and enema agents can be illustrated. Preferably, the pharmaceutical composition of the present invention is orally administered.

The pharmaceutical composition of the present invention comprises a crystal of the compound 1 as an active ingredient.

The pharmaceutical composition of the present invention is prepared using a crystal of the compound 1 and at least one pharmaceutical additive. These pharmaceutical compositions can be formulated by appropriately admixing, diluting or dissolving with appropriate pharmaceutical additives such as excipients, disintegrants, binders, lubricants, diluents, buffers, tonicity agents, preservatives, wetting agents, emulsifying agents, dispersing agents, stabilizing agents, solubilizing agents and the like, according to a known formulation procedure depending upon their dosage forms.

When the pharmaceutical composition of the present invention is used in the treatment, the dosage of the compound 1 is appropriately decided depending on the age, sex, body weight and degree of disorders and treatment of each patient and the like. The daily dose can be divided into one, two, three or four times per day and administered.

The dosage for an adult can be decided within the range of, for example, 0.1 to 1000 mg per day in the case of oral administration. In an embodiment, the oral administration dosage can be decided within the range of 1 to 500 mg per day and is preferably within the range of 10 to 200 mg per day.

The dosage for an adult can be decided at, for example, 0.1 to 1000 mg per day in the case of parenteral administration. In an embodiment, the parenteral administration dosage can be decided within the range of 0.5 to 200 mg per day and is preferably within the range of 1 to 20 mg per day.

In an embodiment, the pharmaceutical composition of the present invention can also be used in combination with any other medicament other than PHD inhibitors. As such other medicaments which can be used in combination for the treatment of inflammatory bowel diseases, for example, 5-ASA, steroids, immunosuppressive agents, TNF-α antibodies, Janus kinase inhibitors and α4β7 integrin antibodies can be illustrated.

When the crystal of the compound 1 of the present invention is used in combination with the other medicament, they can be administered as a formulation comprising these active ingredients or as formulations which are each separately formulated from each active ingredient. When separately formulated, these formulations can be administered separately or concurrently. Furthermore, the dosage of the compound 1 of the present invention can be appropriately reduced depending on the dosage of the other medicament used in combination.

### Measurement of Powder X-ray analysis

For the powder X-ray diffraction, the crystals were ground with a mortar and then measured with a powder X-ray diffraction apparatus SmartLab (Rigaku) by reflection method according to the following conditions.
X-ray source, Wavelength: CuKα rays (CuKα1 and CuKα2), 1.5418Å
Tube voltage, Tube current, Scanning speed: 40 kV, 50 mA, 13° 2(*θ*)/min
Data analysis software: SmartLab Studio II (Rigaku)
Data analysis method (peak definition): Peak position (peak top position, diffraction angles upon irradiation with CuKα1 and CuKα2), peak height (exclude background)

It is common knowledge that the relative intensity of a peak (relative peak height) in powder X-ray diffraction patterns may fluctuate depending on the sample conditions and the measurement conditions. The relative intensity can slightly vary depending on the direction of crystal growth, the size of particles, the measurement conditions or the like and therefore should not be strictly interpreted.

It is publicly known that the 2*θ* values of diffraction patterns in powder X-ray diffraction may slightly fluctuate depending on sample conditions and measurement conditions. In general, the 2*θ* values may fluctuate within a range of about ±0.2 (°). Therefore, the present invention encompasses not only crystals in which the diffraction angles (2*θ* (°)) of peaks in powder X-ray diffraction completely coincide but also crystals in which the diffraction angles (2*θ* (°)) of all or a part of the peaks coincide within a range of ±0.2 (°).

### Thermal Analysis Measurement (Thermogravimetric differential thermal analysis (TG-DTA))

Thermal analysis was performed using a differential thermobalance (type TG8120 or type Thermo plus EVO2, Rigaku) under nitrogen atmosphere according to the following measurement conditions.

### Heating rate: 10°C/min

### Reference material: Aluminium oxide

The temperature at which the mass decrease started and the temperature at which it converged were measured, and the mass change (%) was calculated from the mass difference at the temperatures.

In a TG-DTA measurement diagram, "endotherm" in a DTA curve is represented by the temperature at peak top (peak top) or "extrapolated start temperature". "Extrapolated start temperature" means the intersection between the onset point or the offset point in the DTA curve and extrapolation of the baseline and is also referred to as "extrapolated onset temperature". "Extrapolated start temperature" is a temperature at the starting point of the peak, and it means exothermic or endothermic starting temperature calculated by the extrapolation. The peak top and the extrapolated start temperature in a TG-DTA measurement diagram may slightly fluctuate depending on the measurement conditions. For example, in general, the temperature may fluctuate within a range of ±5°C. Thus, the crystal specified by the above peaks encompasses crystals which coincide within a range of ±5°C.

In the present invention, "around" used in the thermal analysis means a range of ±5°C and preferably means ±2°C.

The crystal of the compound 1 of the present invention can be specified for its crystal form by a subset of the diffraction peaks in the powder X-ray diffraction described in each example. In the present invention, "having a subset of the peaks" means that a crystal includes at least the peaks of the subset as the characteristic peaks.

Further, crystal forms can also be specified by a combination of a subset of the diffraction peaks in the powder X-ray diffraction and physical properties such as heat absorption in TG-DTA measurement of each crystal.

### Examples

The present invention is further illustrated in more detail by way of the following Examples. However, the present invention is not limited thereto.

### Reference Example 1

### 2,6-Dichloro-7-(4-methoxybenzyl)-7H-purine

To a solution of 4-methoxybenzyl chloride (9.40 g) in THF (100 mL) was added sodium iodide (9.74 g) at room temperature. After the reaction mixture was stirred for 1 h, 2,6-dichloropurine (9.45 g) and potassium carbonate (10.37 g) were added. The reaction mixture was stirred at room temperature for 20 h. To the reaction mixture was added water and it was extracted with ethyl acetate. The organic layer was washed with brine, dried over anhydrous magnesium sulfate, and then concentrated under reduced pressure. The obtained residue was purified by column chromatography on silica gel (eluent: ethyl acetate/hexane=37/63-58/42-70/30) to give each 2,6-Dichloro-9-(4-methoxybenzyl)-9H-purine (2.48 g) and title compound (2.22 g). ¹H-NMR (CDCl₃) δ ppm: 3.82 (3H, s), 5.59 (2H, s), 6.80-7.00 (2H, m), 7.05-7.25(2H, m), 8.18 (1H, s).

### Reference Example 2

### 2-Chloro-6-methoxy-7-(4-methoxybenzyl)-7H-purine

To a solution of Reference Example 1 (4.35 g) in THF (70 mL) was added dropwise 28% solution of sodium methoxide in methanol (2.58 g) under ice-cooling under an argon atmosphere. The reaction mixture was stirred at room temperature for 30 minutes. To the reaction mixture was added water and the insoluble material was collected by filtration. The obtained solid was washed with water, and then dried under reduced pressure at 50°C for 6 h to give the title compound (3.47 g).

### Reference Example 3

### Ethyl 1-(6-methoxy-7-(4-methoxybenzyl)-7H-purin-2-yl)-1H-pyrazole-4-carboxylate

To a solution of Reference Example 2 (3.21 g), ethyl 1H-pyrazole-4-carboxylate (1.77 g) and tBuXPhos (0.90 g) in NMP (21 mL) were added tripotassium phosphate (3.35 g) and Pd₂(dba)₃ (0.48 g) under an argon atmosphere. The reaction mixture was stirred at 60°C for 3 h. After the reaction mixture was allowed to cool to room temperature, to the reaction mixture were added water and ethyl acetate, and the insoluble material was removed by passing through Hyflo Super-Cel. After the filtrate was extracted with ethyl acetate, the organic layer was washed with water and brine. The organic layer was passed through amino-silica gel and concentrated under reduced pressure. To the obtained residue was added ethanol and the insoluble material was collected by filtration. The obtained solid was washed with ethanol and diisopropyl ether, and then purified by column chromatography on amino-silica gel (eluent: ethyl acetate/hexane=81/19-100/0) to give the title compound (2.02 g).

### Compound 1

### 1-(7-(4-Methoxybenzyl)-6-oxo-6,7-dihydro-1H-purin-2-yl)-1H-pyrazole-4-carboxylic acid

To a solution of Reference Example 3 (4.48 g) in THF (55 mL) were added water (27 mL) and an aqueous solution of lithium hydroxide (4mol/L, 27 mL). The reaction mixture was stirred at 50°C for 18 h. After the reaction mixture was allowed to cool to room temperature, to the reaction mixture was added 2 mol/L hydrochloric acid (66 mL) and it was stirred at room temperature for 1 h. The resulting insoluble material was collected by filtration. The obtained solid was washed with water, and then dried under reduced pressure at 50°C for 8 h to give the title compound (3.87 g).

**[Table 1]**

| | Structure | Physical data | IC₅₀ (*µ*M) |
|---|---|---|---|
| Compound 1 | 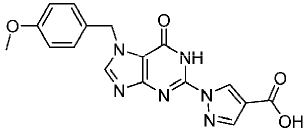 | ¹H-NMR (DMSO-d6) *δ*ppm: 3.72 (3H, s), 5.49 (2H, s), 6.80-7.00 (2H, m), 7.25-7.45 (2H, m), 8.15-8.30 (1H, m), 8.44 (1H, s), 8.80-8.95 (1H, m), 12.60-13.20 (2H, m). MS m/z : 367(M+H)⁺ | 0.44 |

### Test Example 1 PHD2 inhibitory test

### (1) Expression and preparation of human PHD2₁₈₄₋₄₁₈

Human PHD2₁₈₄₋₄₁₈ containing amino acid residues 184 to 418 of the protein represented by CAC42509 (GenBank accession ID) was expressed and prepared by the following method.

An expression construct of human PHD2₁₈₄₋₄₁₈ containing N-terminal histidine tag was introduced into pET-30a (+) vector, and the sequence was confirmed. This vector was introduced into BL21 (DE3) strain and cultured at 37°C in LB medium containing antibiotics. After culturing, a cell lysis solution was added to the cells, and then the cells were disrupted and suspended by sonication. The disrupted suspension was centrifuged and the supernatant was purified by Ni column to give human PHD2₁₈₄₋₄₁₈.

### (2) Test methods

Human HIF-1α₅₅₆₋₅₇₄ (FITC-labeled HIF-1α₅₅₆₋₅₇₄), containing FITC-Ahx at the N-terminal of HIF-1α₅₅₆₋₅₇₄ containing amino acid residues 556 to 574 (partial peptide) of human HIF-1α was used as a substrate. Using FITC-labeled HIF-1α₅₅₆₋₅₇₄, the competitive inhibition between 2-oxoglutarate and the test compound (PHD inhibitor) was evaluated based on the change in fluorescence polarization of FITC-labeled HIF-1α₅₅₆₋₅₇₄ by the following method.

An enzyme (human PHD2₁₈₄₋₄₁₈) and the substrate were diluted with an assay buffer (pH 7.4) containing 10 mM HEPES, 150 mM NaCl, 10µM MnCl₂-4H₂O, 2µM 2-oxoglutarate and 0.05% Tween-20. The test compound was diluted with DMSO. The test compound and human PHD2₁₈₄₋₄₁₈ were added to the 384-well plate (Corning, black, opaque bottom) in advance. The reaction was started by the addition of FITC-labeled HIF-1α₅₅₆₋₅₇₄. After incubating at 37°C for 60 minutes, fluorescence polarization (excitation wavelength: 470 nm, fluorescence wavelength: 530 nm) was measured by PHERAstar FSX (BMG Labtech). The fluorescence polarization of each well was measured, and the human PHD2 binding inhibitory activity of the test compound was calculated based on the value of the test substance-free group.

### (3) Results

As shown in table 1, the compound 1 of the present invention inhibited binding between PHD2 and HIF-1α. Thus, it was demonstrated that the compound 1 of the present invention is useful as a PHD2 inhibitor.

### Test Example 2 Therapeutic effect in colitis model

### (1) TNBS induced colitis model rat

It is known that inflammation is locally caused in the large intestine when TNBS is administered into the large intestine of a rat and that the intestinal permeability is then increased due to breakdown of barrier function in the intestines. Thus, the suppressive effect on the intestinal permeability based on oral administration of the test compound was evaluated as an indicator of medicinal efficacy.

### (2) Test methods

SD male rats: 8-week-old SLC (Japan SLC) were used. Under pentobarbital anesthesia, 300 µL of TNBS (28 mg/mL) which was prepared by 50% ethanol was administered at a point 8 cm from the anus in the large intestine to cause inflammation. To the solvent-treated group was administrated 300 µL of 50% ethanol. Animals were fasted for 48 hours prior to administration of TNBS. The test compound (3 mg/kg) prepared by 0.05% methylcellulose solution was orally administered once a day from the next day, and it was administered for a total of 3 days. After administering for 3 days, 50 mg/kg FITC was orally administered 4 hours after the administration of the test compound. Blood samples were collected from jugular vein under isoflurane anesthesia after 4 hours. The serum was centrifuged and fluorescence intensity was detected by PHERAstar FSX(BMG Labtech) to measure the concentration of FITC permeating into circulating blood through the mesentery. The suppressive rate on the intestinal permeability of the test compound was calculated based on the value of the test substance-free group as 0 and the value of the TNBS-untreated group as 100.

### (3) Results

The suppressive rate (%, mean) on the intestinal permeability of the test compound (Inhibition) is shown below.

**[Table 2]**

| | Inhibition (%) |
|---|---|
| Compound 1 | 83 |

The intestinal permeability of FITC, which was increased due to the administration of TNBS, was suppressed by the administration of the compound 1 of the present invention. Thus, it was demonstrated that the compound 1 of the present invention is useful as an agent for the treatment of inflammatory bowel diseases.

### Test Example 3 Concentration of compounds in large intestine tissue

### (1) Rat PK study

The test compound (3 mg/kg/5 mL) prepared with 0.05% methylcellulose was orally administered to non-fasted rats (SD, 8-week-old, male, Japan SLC). Blood samples were collected from jugular vein at 0.25, 0.5, 1, 2, 4, 6 and 8 hours after the administration. Laparotomy was performed under isoflurane anesthesia and large intestine was isolated. The collected distal large intestine (about 5 cm) was cut open, and then the large intestine extracted was washed with saline on a dish. After washing, the large intestine was minced with small scissors. About 150 mg thereof was moved to a tube. To the tube was added 100 µL of saline, and the mixture was homogenized using shake master (1000 rpm × 30 minutes). Samples were prepared by the addition with quadruple volume of saline as the final volume. The concentrations of the test compound in the large intestine tissue and the plasma were measured through a quantitative analysis using liquid chromatography-mass spectrometry (LC/MS).

As the comparative example, a compound described as example 55 in US2015/0239889 (WO2014/030716) (Compound A) was used.

### (2) Concentration of compounds in large intestine tissue and plasma

As shown in the following table, it was demonstrated that the compound 1 of the present invention has a higher concentration in the large intestine tissue than the concentration in the plasma. Accordingly, the compound 1 of the present invention is a PHD2 inhibitor that acts specifically on the large intestine tissue.

**[Table 3]**

| | Cmax | AUC | Plasma | Colon | C/P |
|---|---|---|---|---|---|
| Compound 1 | 10 | 1,095 | <1 | 209 | >209 |
| Compound A | 281 | 79,955 | 74 | <50 | <0.7 |

Symbols in the table have the following meanings.
Compound A: comparative example
Cmax: maximum plasma concentration of the test compound in the case of oral administration (ng/mL)
AUC: area under the plasma test compound concentration-time curve (ng^{∗}min/mL)
Plasma: plasma test compound concentration after 8 hours (ng/mL)
Colon: concentration of the test compound in the large intestine tissue after 8 hours (ng/g)
C/P: ratio of the above Colon and Plasma

### Example 1-1: crystal form I of the compound 1

A mixture of Reference Example 3 (4.90 g), THF (40 mL) , an aqueous solution of lithium hydroxide (4mol/L, 30 mL) and water (15 mL) was stirred at 60°C for 4 hours. The reaction mixture was heated to 50°C, and 2 mol/L hydrochloric acid was added dropwise by small portions to reach pH 2. When 2 mol/L hydrochloric acid (38 mL) was added dropwise, the reaction mixture was filtered through Celite. The Celite was washed with water (20mL), and the filtrate was combined with the first filtrate. To the obtained filtrate was added dropwise 2 mol/L hydrochloric acid (72 mL in total including the above 38 mL). After stirring the obtained suspension for 1 hour at 50°C and 12 hours at room temperature, the slurry was filtered under suction. The obtained solid was washed with water (10 mL) twice and then water (30 mL) three times. The obtained solid was dried under reduced pressure at 50°C to give the crystal form I of the compound 1 (4.33 g).

The powder X-ray diffraction for the crystal form I of the compound 1 was measured. The diffraction angles (2*θ* (°)) of major diffraction peaks and the relative intensities (Rel. Den. (%)) of the diffraction peaks are shown in table 4.

**[Table 4]**

| 2 *θ* (° ) | Rel. Den. (%) | 2 *θ* (° ) | Rel. Den. (%) |
|---|---|---|---|
| 6. 5 | 100 | 16. 2 | 37 |
| 9. 7 | 14 | 17. 5 | 29 |
| 13. 5 | 69 | 19. 9 | 18 |
| 15. 9 | 11 | | |

For the identification of the crystal form I of the compound 1, for example, a subset of the diffraction peaks selected from the group consisting of the following [1-1-1] to [1-1-6] can be used:
[1-1-1] peaks of 6.5±0.2 and 13.5±0.2;
[1-1-2] peaks of 6.5±0.2 and 16.2±0.2;
[1-1-3] peaks of 6.5±0.2, 13.5±0.2 and 16.2±0.2;
[1-1-4] peaks of 6.5±0.2, 13.5±0.2, 16.2±0.2 and 17.5±0.2;
[1-1-5] peaks of 6.5±0.2, 9.7±0.2, 13.5±0.2, 16.2±0.2 and 17.5±0.2; and
[1-1-6] peaks of 6.5±0.2, 9.7±0.2, 13.5±0.2, 16.2±0.2 and 17.5±0.2 and 19.9±0.2.

Thermal analysis for the crystal form I of the compound 1 was performed. Endotherm: around 300°C (peak top, extrapolated start temperature around 298°C) Mass decrease: around 30°C to 280°C (0.7%)

### Example 1-2: crystal form II of the compound 1

A suspension of the compound 1 (10 mg), 1,4-Dioxane (0.9 mL), water (0.1 mL) and 2 mol/L sodium hydroxide aqueous solution (27.5 µL) was stirred at 60°C. After dissolution, the solution was filtered. To the filtrate was added 2 mol/L hydrochloric acid (41 µL) at 40°C. The mixture was stirred for 2 hours at the same temperature, then stirred for 60 hours at room temperature. The slurry was filtered under suction to give the crystal form II of the compound 1. The obtained crystal form II was used as seed crystal for next operation.

A suspension of the compound 1 (150 mg), 1,4-Dioxane (3 mL), water (1.5 mL) and 2 mol/L sodium hydroxide aqueous solution (413 µL) was stirred at 60°C. After dissolution, the solution was filtered. To the filtrates was added 2 mol/L hydrochloric acid (413 µL) at 40°C. At the same temperature, the seed crystal (1 mg) was added, and the solution was stirred for 1 hour. To the mixture was added 2 mol/L hydrochloric acid (207 µL) at room temperature, and the mixture was stirred for 2 hours. The slurry was filtered under suction, and the obtained solid was washed with water (5 mL) twice. The obtained solid was dried under reduced pressure at 50°C to give the crystal form II of the compound 1 (118 mg).

The powder X-ray diffraction for the crystal form II of the compound 1 was measured. The diffraction angles (2*θ* (°)) of major diffraction peaks and the relative intensities (Rel. Den. (%)) of the diffraction peaks are shown in table 5.

**[Table 5]**

| 2 *θ* (° ) | Rel. Den. (%) | 2 *θ* (° ) | Rel. Den. (%) |
|---|---|---|---|
| 5. 1 | 100 | 16. 4 | 21 |
| 8. 3 | 11 | 16. 9 | 50 |
| 8. 6 | 12 | 26. 7 | 16 |
| 10. 8 | 15 | | |

For the identification of the crystal form II of the compound 1, for example, a subset of the diffraction peaks selected from the group consisting of the following [1-2-1] to [1-2-6] can be used:
[1-2-1] peaks of 5.1±0.2 and 16.9±0.2;
[1-2-2] peaks of 5.1±0.2 and 10.8±0.2;
[1-2-3] peaks of 5.1±0.2, 16.4±0.2 and 16.9±0.2;
[1-2-4] peaks of 5.1±0.2, 10.8±0.2, 16.4±0.2 and 16.9±0.2;
[1-2-5] peaks of 5.1±0.2, 8.6±0.2, 10.8±0.2, 16.4±0.2 and 16.9±0.2; and
[1-2-6] peaks of 5.1±0.2, 8.6±0.2, 10.8±0.2, 16.4±0.2, 16.9±0.2 and 26.7±0.2.

Thermal analysis for the crystal form II of the compound 1 was performed. Endotherm: around 301°C (peak top, extrapolated start temperature around 300°C) Mass decrease: around 30°C to 290°C (0.6%)

### Example 1-3: crystal form III of the compound 1

A suspension of the compound 1 (20 mg), methanol (0.4 mL), water (0.2 mL) and triethylamine (15 µL) was stirred at 60°C. After dissolution, acetic acid (10 µL) was added to the solution, and the solution was stirred for 5 days at the same temperature. The generated crystals in the mixture were collected to give the crystal form III of the compound 1. The obtained crystal form III was used as seed crystal for next operation.

A mixture of the crystal form I of the compound 1 (0.2 g), methanol (8 mL) and the seed crystal of the crystal form III (1 mg) was stirred at 75°C for about 2 hours. The mixture was stirred at 65°C, and to the mixture was added the seed crystal (1 mg), and the mixture was stirred for 2 days. The suspension was cooled to room temperature and filtered under suction. The obtained solid was washed with methanol (2 mL) and dried under reduced pressure at 50°C to give the crystal form III of the compound 1 (157 mg).

The powder X-ray diffraction for the crystal form III of the compound 1 was measured. The diffraction angles (2*θ* (°)) of major diffraction peaks and the relative intensities (Rel. Den. (%)) of the diffraction peaks are shown in table 6.

**[Table 6]**

| 2 *θ* (° ) | Rel. Den. (%) | 2 *θ* (° ) | Rel. Den. (%) |
|---|---|---|---|
| 5. 8 | 100 | 17. 4 | 30 |
| 8. 4 | 25 | 17. 7 | 31 |
| 13. 6 | 32 | 25. 4 | 37 |
| 17. 0 | 23 | 25. 8 | 22 |

For the identification of the crystal form III of the compound 1, for example, a subset of the diffraction peaks selected from the group consisting of the following [1-3-1] to [1-3-6] can be used:
[1-3-1] peaks of 5.8±0.2 and 17.7±0.2;
[1-3-2] peaks of 5.8±0.2 and 25.4±0.2;
[1-3-3] peaks of 5.8±0.2, 17.7±0.2 and 25.4±0.2;
[1-3-4] peaks of 5.8±0.2, 13.6±0.2, 17.7±0.2 and 25.4±0.2;
[1-3-5] peaks of 5.8±0.2, 8.4±0.2, 13.6±0.2, 17.7±0.2 and 25.4±0.2; and
[1-3-6] peaks of 5.8±0.2, 8.4±0.2, 13.6±0.2, 17.0±0.2, 17.4±0.2, 17.7±0.2, 25.4±0.2 and 25.8±0.2.

Thermal analysis for the crystal form III of the compound 1 was performed. Endotherm: around 294°C (peak top, extrapolated start temperature around 291°C) Mass decrease: around 30°C to 280°C (0.2%)

### Example 1-4: crystal form IV of the compound 1

The compound 1 (600 mg) was suspended in 3 mL of methanol. To the suspension was added 1 mol/L sodium hydroxide aqueous solution (3.28 mL) and then water (0.9 mL) at room temperature. After dissolution, to the solution was added 1 mol/L hydrochloric acid (1.64 mL) at room temperature. Precipitation was immediately observed. The mixture was stirred at 50°C for 1 hour, and to the mixture was added methanol (3 mL). The mixture was further stirred at 50°C for 1 hour, and to the mixture was added methanol (0.6 mL). The mixture was stirred at room temperature for 1 hour. The slurry was filtered under suction. The obtained solid was washed with 30% water-containing methanol, dried under reduced pressure for 2 hours at 40°C to give the crystal form I of the sodium salt of the compound 1 (471 mg).

The crystal form I of the sodium salt of the compound 1 (59 mg) was suspended in the 1st fluid of the dissolution test in the Japanese Pharmacopoeia (6 mL), and the suspension was shaken and stirred for 15 hours at 37°C. The solid was collected by filtration, and the obtained solid was dried under reduced pressure for 2 hours at 40°C to give the crystal form IV of the compound 1 (47 mg).

The powder X-ray diffraction for the crystal form IV of the compound 1 was measured. The diffraction angles (2*θ* (°)) of major diffraction peaks and the relative intensities (Rel. Den. (%)) of the diffraction peaks are shown in table 7.

**[Table 7]**

| 2 *θ* (° ) | Rel. Den. (%) | 2 *θ* (° ) | Rel. Den. (%) |
|---|---|---|---|
| 5. 0 | 100 | 16. 3 | 28 |
| 5. 6 | 65 | 19. 6 | 28 |
| 14. 0 | 24 | 20. 6 | 35 |
| 15. 2 | 32 | 26. 4 | 73 |

For the identification of the crystal form IV of the compound 1, for example, a subset of the diffraction peaks selected from the group consisting of the following [1-4-1] to [1-4-5] can be used:
[1-4-1] peaks of 5.0±0.2 and 5.6±0.2;
[1-4-2] peaks of 5.0±0.2, 15.2±0.2 and 26.4±0.2;
[1-4-3] peaks of 5.0±0.2, 5.6±0.2, 15.2±0.2 and 26.4±0.2;
[1-4-4] peaks of 5.0±0.2, 5.6±0.2, 15.2±0.2, 16.3±0.2, 20.6±0.2 and 26.4±0.2; and
[1-4-5] peaks of 5.0±0.2, 5.6±0.2, 14.0±0.2, 15.2±0.2, 16.3±0.2, 19.6±0.2, 20.6±0.2 and 26.4±0.2.

Thermal analysis for the crystal form III of the compound 1 was performed. Endotherm: around 235°C (peak top), around 301°C (peak top, extrapolated start temperature around 299°C)
Mass decrease: around 25°C to 100°C (0.5%)

### Test Example 4: Solid stability test

Physicochemical stability and chemical stability for crystals of compound 1 (crystal forms I to IV) were examined.

### (1) Method

Physicochemical stability: Each crystal was stored under open condition at 40°C. The powder X-ray diffraction of the sample was measured at the initial point and 1 month later, and the change in crystal form was confirmed. The change in properties was also observed at the same time.
Chemical stability: Each crystal was stored under open condition at 40°C. The mass of the compound 1 was measured under the following HPLC measurement conditions at the initial point and 1 month later.

### [HPLC conditions]

Detector: Ultraviolet and visible absorptiometer / wavelength: 225 nm
Column: L-column2 ODS, 3 µm, 4.6×150 mm (Chemicals Evaluation and Research Institute)
Column temperature: Constant temperature around 40°C
Flow rate: 1.0 mL/min
Mobile phase A: A solution which was prepared with phosphoric acid to pH 2.3 after dissolving potassium dihydrogen phosphate in water to 20 mmol/L
Mobile phase B: Acetonitrile
Mobile phase ratios
0 to 30 minutes: mobile phase A/mobile phase B = 81/19
30 to 50 minutes: mobile phase A/mobile phase B = 81/19 to 25/75
50 to 55 minutes: mobile phase A/mobile phase B = 25/75
55 to 55.01 minutes: mobile phase A/mobile phase B = 25/75 to 81/19
55.01 to 75 minutes: mobile phase A/mobile phase B = 81/19
Amount of injection: 5 µL
Sample cooler: 4°C
Dissolution solvent: Mixed solution (mobile phase A/acetonitrile=75/25)
Sample solution: Sample was dissolved in dissolution solvent and adjusted to about 1.0 mg/mL

Excluding the peaks derived from the blank, the peak areas of the compound 1 and an analogous substance were measured by automatic integration, and the mass of the compound 1 was calculated by area normalization method. Further, the amount of the mass change of the compound 1 after the storage was calculated.

### (2) Results

In the storage under open condition at 40°C, the crystals of the compound 1 were physicochemically stable with virtually no changes in crystal form and in properties. In addition, the crystals of the compound 1 were chemically stable with virtually no decrease of the amount of the compound 1 (Table 8).

Therefore, it was demonstrated that the crystal of the compound 1 of the present invention has good physical properties as a drug substance.

**[Table 8]**

| Crystal Form | Amount of the mass change of the compound l | Crystal form change | Properties |
|---|---|---|---|
| Crystal form I of the compound 1 | -0. 05% | No change | White powder, no change |
| Crystal form II of the compound l | 0. 03% | No change | White powder, no change |
| Crystal form III of the compound 1 | 0. 00% | No change | White powder, no change |
| Crystal form IV of the compound 1 | 0. 00% | No change | White powder, no change |

### [Industrial Applicability]

The crystal of the compound 1 of the present invention has good physical properties as a drug substance and is useful as an agent for the treatment of an inflammatory bowel disease.

## Claims

1. A crystal of the compound represented by the following formula (I):

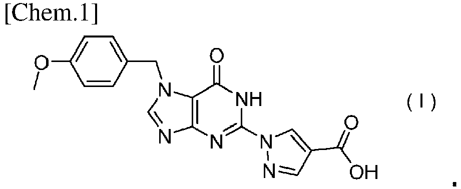

2. The crystal according to claim 1 which is a crystal of the compound having a subset of the peaks at diffraction angles (2*θ* (°)) selected from the group consisting of the following (i) to (iv) in a powder X-ray diffraction:
(i) peaks of 6.5±0.2 and 13.5±0.2;
(ii) peaks of 5.1±0.2 and 16.9±0.2;
(iii) peaks of 5.8±0.2 and 17.7±0.2; and
(iv) peaks of 5.0±0.2 and 5.6±0.2.

3. The crystal according to claim 1 which is a crystal of the compound having a subset of the peaks at diffraction angles (2*θ* (°)) selected from the group consisting of the following (i) to (iv) in a powder X-ray diffraction:
(i) peaks of 6.5±0.2 and 16.2±0.2;
(ii) peaks of 5.1±0.2 and 10.8±0.2;
(iii) peaks of 5.8±0.2 and 25.4±0.2; and
(iv) peaks of 5.0±0.2, 15.2±0.2 and 26.4±0.2;

4. The crystal according to claim 1 which is a crystal of the compound having peaks at diffraction angles (2*θ* (°)) of 5.8±0.2, 8.4±0.2, 13.6±0.2, 17.0±0.2, 17.4±0.2, 17.7±0.2, 25.4±0.2 and 25.8±0.2 in a powder X-ray diffraction.

5. A pharmaceutical composition comprising the crystal according to any one of claims 1 to 4 and a pharmaceutical additive.

6. The pharmaceutical composition according to claim 5 which is a pharmaceutical composition for use in the treatment of an inflammatory bowel disease.

7. The pharmaceutical composition according to claim 6, wherein the inflammatory bowel disease is ulcerative colitis or Crohn's disease.
